(19) Europäisches Patentamt
European Patent Office
Office européen des brevets

(11) Veröffentlichungsnummer : **0 039 455**
**B1**

(12) # EUROPÄISCHE PATENTSCHRIFT

(45) Veröffentlichungstag der Patentschrift : **04.07.84**

(51) Int. Cl.³ : **C 07 C101/447**, A 61 K 7/13, C 07 C 93/14

(21) Anmeldenummer : **81103090.7**

(22) Anmeldetag : **24.04.81**

(54) **Neue Kupplerkomponenten für Oxidationshaarfarben, deren Herstellung sowie diese enthaltende Haarfärbemittel.**

(30) Priorität : **02.05.80 DE 3016881**

(43) Veröffentlichungstag der Anmeldung : **11.11.81 Patentblatt 81/45**

(45) Bekanntmachung des Hinweises auf die Patenterteilung : **04.07.84 Patentblatt 84/27**

(84) Benannte Vertragsstaaten : **AT BE CH DE FR GB IT LI LU NL SE**

(56) Entgegenhaltungen :
**EP-A- 0 012 965**
**DE-A- 2 737 138**
**Chemical Abstracts, Band 91, Nr. 13, 24 September 1979 Columbus, Ohio, USA WILLIAMS et al. "The Action of DL-2,4-Diaminobutyric Acid on the Cataleptogenic Effects of Pilocarpine and alpha-Flupenthixol in Rats" Seite 62, linke Spalte, Abstract Nr. 102177j**
**Die Akte enthält technische Angaben, die nach dem Eingang der Anmeldung eingereicht wurden und die nicht in dieser Patentschrift enthalten sind.**

(73) Patentinhaber : **Henkel Kommanditgesellschaft auf Aktien**
**Postfach 1100 Henkelstrasse 67**
**D-4000 Düsseldorf-Holthausen (DE)**

(72) Erfinder : **Konrad, Günther, Dr.**
**Feuerbachweg 12**
**D-4010 Hilden (DE)**
Erfinder : **Maak, Norbert, Dr.**
**Liebigstrasse 18**
**D-4040 Neuss (DE)**
Erfinder : **Lieske, Edgar**
**Hunsrückenstrasse 40**
**D-4000 Düsseldorf (DE)**

Anmerkung : Innerhalb von neun Monaten nach der Bekanntmachung des Hinweises auf die Erteilung des europäischen Patents im Europäischen Patentblatt kann jedermann beim Europäischen Patentamt gegen das erteilte europäische Patent Einspruch einlegen. Der Einspruch ist schriftlich einzureichen und zu begründen. Er gilt erst als eingelegt, wenn die Einspruchsgebühr entrichtet worden ist (Art. 99(1) Europäisches Patentübereinkommen).

Jouve, 18, rue St-Denis, 75001 Paris, France

EP 0 039 455 B1

**Beschreibung**

Oxidationsfarben, wie sie durch oxidative Kupplung einer Entwicklerkomponente mit einer Kupplerkomponente entstehen, spielen wegen ihrer intensiven Farbnuancen und ihrer sehr guten Echtheitseigenschaften eine bevorzugte Rolle beim Färben von Haaren. Als Entwicklersubstanzen werden üblicherweise Stickstoffbasen wie p-Phenylendiaminderivate, Diaminopyridine, 4-Aminopyrazolonderivate oder heterocyclische Hydrazone eingesetzt. Als Kupplerkomponenten dienen m-Phenylendiaminderivate, Phenole, Naphthole, Resorcinderivate und Pyrazolone.

Aus DE-OS 2 737 138 sind 2.4-Diaminophenolether als Kupplerverbindungen für Oxidationshaarfärbemittel bekannt, die bezüglich der Thermostabilität und der toxikologischen Eigenschaften nicht voll befriedigen.

Von brauchbaren Komponenten für Oxidationshaarfarbstoffe wird verlangt, daß sie bei der oxidativen Kupplung mit den vorgesehenen Kuppler- oder Entwicklersubstanzen die gewünschten Farbnuancen in ausreichender Intensität ausbilden. Sie müssen ferner ein ausreichendes bis sehr gutes Aufziehvermögen auf menschliches Haar besitzen. Darüber hinaus sollen sie toxikologisch und dermatologisch unbedenklich sein.

Bei der Suche nach brauchbaren Oxidationsfarbstoffen bestand daher die Aufgabe, geeignete Komponenten aufzufinden, welche die oben genannten Voraussetzungen in optimaler Weise erfüllen.

Es wurde gefunden, daß man zu Oxidationshaarfarben gelangt, die den gestellten Anforderungen in besonders hohem Maße gerecht werden, wenn man als Kupplerkomponenten die nachstehend beschriebenen neuen Verbindungen in Kombination mit üblichen Entwicklersubstanzen verwendet.

Gegenstand der Erfindung sind m-Phenylendiaminderivate der Formel I,

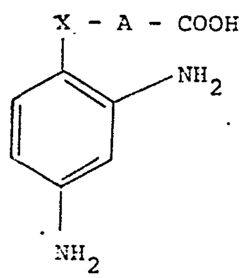

(I)

in der X ein Sauerstoffatom bedeutet oder nicht vorhanden ist, während A für einen Alkylenrest mit 1 bis 4 Kohlenstoffatomen steht.

Die Erfindung betrifft weiterhin ein Verfahren zur Herstellung der Verbindungen der Formel I, bei dem man Verbindungen der Formel II

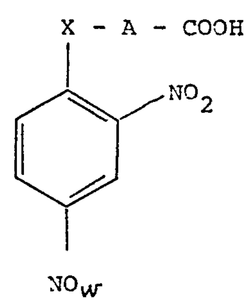

(II)

in der X und A die für die Formel I angegebene Bedeutung haben, in an sich bekannter Weise in Gegenwart eines Katalysators mit Wasserstoff umsetzt.

Gegenstand der Erfindung sind schließlich auch Haarfärbemittel auf Basis von Oxidationsfarbstoffen mit einem Gehalt an Verbindungen der Formel I und/oder deren Ammonium-, Alkali- oder Erdalkalisalzen als Kupplerkomponenten und den in Oxidationshaarfarben üblichen Entwicklersubstanzen.

Bei ihrem Einsatz als Kupplerkomponenten liefern die erfindungsgemäßen Verbindungen der Formel I mit den im allgemeinen für die Oxidationshaarfärbung verwendeten Entwicklersubstanzen sehr intensive Farbtöne im blauen und violetten Bereich und stellen somit eine wesentliche Bereicherung der oxidativen Haarfärbemöglichkeiten dar. Darüber hinaus zeichnen sich die erfindungsgemäßen m-Phenylendiaminderivate durch sehr gute Echtheitseigenschaften der damit erzielten Färbungen, durch gute Löslichkeit in Wasser, gute Lagerbeständigkeit sowie durch toxikologische und dermatologische Unbedenklichkeit aus.

Haarfärbemittel auf Basis von Oxidationsfarbstoffen mit einem Gehalt an m-Phenylendiaminderivaten der Formel I und/oder deren Ammonium-, Alkali- und Erdalkalisalzen und den in Oxidationshaarfarben üblichen Entwicklersubstanzen stellen demnach besonders wertvolle Kompositionen auf dem Gebiet der Oxidationshaarfarben dar.

Die als Ausgangsmaterial für die Herstellung der m-Phenylendiaminderivate der Formel I dienenden 2,4-Dinitrophenylverbindungen stellen eine bekannte Substanzklasse dar ; sie können nach oder in Analogie zu literaturbekannten Verfahren hergestellt werden. In diesem Zusammenhang wird auf Gazz. Chim. Ital. 22 I (1982), S. 242, Ber. *13* (1880), S. 1680, Ber. *40* (1907), S. 1596, Ber. *42* (1908), S. 1310 und J. Med. Chem. *11* (1968) S. 424 verwiesen.

Die Reduktion der Verbindungen der Formel II mit Wasserstoff erfolgt in Gegenwart von Hydrierkatalysatoren, wie z. B. Palladium, das auf Aktivkohle niedergeschlagen ist, im allgemeinen ohne äußere Wärmezufuhr.

Als Beispiele für erfindungsgemäße m-Phenylendiaminderivate sind 2,4-Diaminophenylessigsäure, 2,4-Diaminophenoxyessigsäure, 3-(2,4-Diaminophenyl)-propionsäure, 3-(2,4-Diaminophenoxy)-propionsäure, 2-(2,4-Diaminophenyl)-buttersäure, 4-(2,4-Diaminophenyl)-buttersäure und 4-(2,4-Diaminophenoxy)-buttersäure zu nennen.

Als Beispiele für Entwicklerkomponenten, die in den erfindungsgemäßen Haarfärbemitteln eingesetzt werden können, sind primäre aromatische Amine mit einer weiteren in p-Stellung befindlichen funktionellen Gruppe wie p-Phenylendiamin, p-Toluylendiamin, p-Aminophenol, N-Methyl-p-phenylendiamin, N,N-Dimethyl-p-phenylendiamin, N,N-Diethyl-2-methyl-p-phenylendiamin, N-Ethyl-N-hydroxyethyl-p-phenylendiamin, Chlor-p-phenylendiamin, N,N-Bis-hydroxyethylamino-p-phenylendiamin, Methoxy-p-phenylendiamin, 2-Chlor-, 2,6-Dichlor-p-phenylendiamin, 2-Chlor-6-brom-p-phenylendiamin, 2-Chlor-6-methyl-p-phenylendiamin, 6-Methoxy-3-methyl-p-phenylendiamin, andere Verbindungen der genannten Art, die weiterhin eine oder mehrere funktionelle Gruppen wie OH-Gruppen, $NH_2$-Gruppen, NHR-Gruppen, $NR_2$-Gruppen, wobei R einen Alkyl- oder Hydroxyalkylrest mit 1-4 Kohlenstoffatomen darstellt, enthalten, ferner Diaminopyridinderivate, heterocyclische Hydrazonderivate wie 1-Methyl-pyrrolidon-(2)-hydrazon, 4-Aminopyrazolonderivate wie 4-Amino-1-phenyl-3-carbamoylpyrazolon-5, N-Butyl-N-sulfobutyl-p-phenylendiamin, Tetraaminopyrimidine wie 2,4,5,6-Tetraaminopyrimidin, 2-Methylamino-4,5,6-triaminopyrimidin, 4,5-Diamino-2,6-bismethyl-aminopyrimidin, 2,5-Diamino-4-diethylamino-6-methyl-aminopyrimidin, 2,4,5-Triamino-6-dimethylaminopyrimidin, 2,4,5-Triamino-6-piperidino-pyrimidin, 2,4,5-Triamino-6-anilino-pyrimidin, 2,4,5-Triamino-6-morpholino-pyrimidin, 2,4,5-Triamino-6-ß-hydroxyethylaminopyrimidin anzuführen.

In den erfindungsgemäßen Haarfärbemitteln werden die Entwicklerkomponenten im allgemeinen in etwa molaren Mengen, bezogen auf die verwendeten Kupplersubstanzen, eingesetzt. Wenn sich auch der molare Einsatz als zweckmäßig erweist, so ist es jedoch nicht nachteilig, wenn die Kupplerkomponente in einem gewissen Überschuß oder Unterschuß zum Einsatz gelangt.

Es ist ferner nicht erforderlich, daß die Entwicklerkomponente und die Kupplersubstanz einheitliche Produkte darstellen, vielmehr können sowohl die Entwicklerkomponente als auch die Kupplersubstanz Gemische der vorstehend genannten entsprechenden Verbindungen darstellen.

Darüber hinaus können die erfindungsgemäßen Haarfärbemittel andere bekannte und übliche Kupplersubstanzen sowie auch gegebenenfalls übliche direktziehende Farbstoffe im Gemisch enthalten, falls dies zur Erzielung gewisser Farbnuancen erforderlich ist.

Die oxidative Kupplung, das heißt die Entwicklung der Färbung, kann grundsätzlich wie bei anderen Oxidationshaarfarbstoffen auch, durch Luftsauerstoff erfolgen. Zweckmäßigerweise werden jedoch chemische Oxidationsmittel eingesetzt. Als solche kommen insbesondere Wasserstoffperoxid oder dessen Anlagerungsprodukte an Harnstoff, Melamin und Natriumborat sowie Gemische aus derartigen Wasserstoffperoxidanlagerungsverbindungen mit Kaliumperoxiddisulfat in Betracht.

Die erfindungsgemäßen Haarfärbemittel werden für den Einsatz in entsprechende kosmetische Zubereitungen wie Cremes, Emulsionen, Gele oder auch einfache Lösungen eingearbeitet und unmittelbar vor der Anwendung auf dem Haar mit einem der genannten Oxidationsmittel versetzt. Die Konzentration derartiger färberischer Zubereitungen an Kuppler-Entwicklerkombination beträgt 0,2 bis 5 Gewichtsprozent, vorzugsweise 1 bis 3 Gewichtsprozent. Zur Herstellung von Cremes, Emulsionen oder Gelen werden die Farbstoffkomponenten mit den für derartige Präparationen üblichen weiteren Bestandteilen gemischt. Als solche zusätzlichen Bestandteile sind zum Beispiel Netz- oder Emulgiermittel vom anionischen oder nichtionogenen Typ wie Alkylbenzolsulfonate, Fettalkoholsulfate, Alkylsulfonate, Fettsäurealkanolamide, Anlagerungsprodukte von Ethylenoxid an Fettalkohole, Verdickungsmittel wie Methylcellulose, Stärke, höhere Fettalkohole, Paraffinöl, Fettsäuren, ferner Parfümöle und Haarpflegemittel wie Pantothensäure und Cholesterin zu nennen. Die gennanten Zusatzstoffe werden dabei in den für diese Zwecke üblichen Mengen eingesetzt, wie zum Beispiel Netz- und Emulgiermittel in Konzentrationen von 0,5 bis 30 Gewichtsprozent und Verdickungsmittel in Konzentrationen von 0,1 bis 25 Gewichtsprozent, jeweils bezogen auf die gesamte Zubereitung.

Die Anwendung der erfindungsgemäßen Haarfärbemittel kann, unabhängig davon, ob es sich um eine Lösung, eine Emulsion, eine Creme oder ein Gel handelt, im schwach sauren, neutralen oder insbesondere alkalischen Milieu bei einem pH-Wert von 8 bis 10 erfolgen. Die Anwendungstemperaturen bewegen sich dabei im Bereich von 15 bis 40 °C. Nach einer Einwirkungsdauer von circa 30 Minuten wird das Haarfärbemittel vom zu färbenden Haar durch Spülen entfernt. Hernach wird das Haar mit einem milden Shampoo nachgewaschen und getrocknet.

Mit den erfindungsgemäßen Haarfärbemitteln läßt sich unter Einsatz unterschiedlicher Entwicklerkomponenten und Kupplersubstanzen ein breites Spektrum an Rotbis Dunkelbrauntönen abdecken. Die erzielten Färbungen haben gute Licht-, Wasch- und Reibechtheitseigenschaften und lassen sich leicht mit Reduktionsmitteln wieder abziehen.

Die nachfolgen den Beispiele sollen den Erfindungsgegenstand näher erläutern, ohne ihn jedoch hierauf zu beschränken.

## Beispiele

Zunächst wird die Herstellung der in den folgenden Beispielen für die verschiedenen Ausfärbungen als Kupplerkomponenten eingesetzten erfindungsgemäßen Natriumsalze der 2,4-Diaminophenylessigsäure (K 1) und der 2,4-Diaminophenoxyessigsäure (K 2) beschrieben.

A. Natriumsalz der 2,4-Diaminophenylessigsäure (K 1)

5 g 2,4-Dinitrophenylessigsäure wurden in einer Lösung von 1,9 g Natriumhydrogencarbonat in 200 ml Wasser gelöst und in Gegenwart von 0,3 g Palladium/Kohle bei Raumtemperatur mit Wasserstoff reduziert. Nach dem Ende der Wasserstoffaufnahme wurde der Katalysator abfiltriert und das Filtrat in einem Rotationsverdampfer bei einer Badtemperatur von maximal 30 °C eingedampft. Es wurden 3,5 g 2,4-Diaminoessigsäurenatriumsalz erhalten.

Analyse : $C_8H_9N_2O_2Na + 1,5\ H_2O$
berechnet : 5,62 % H ; 10,69 % Na
gefunden : 5,30 % H ; 10,7 % Na
IR-Spektrum (KBr) : Carbonylbanden bei 1 620 und 1 423 $cm^{-1}$.

B. Natriumsalz der 2,4-Diaminophenoxyessigsäure (K 2)

5 g 2,4-Dinitrophenoxyessigsäure wurden in einer Lösung von 1,8 g Natriumhydrogencarbonat in 200 ml Wasser gelöst und in Gegenwart von 0,3 g Palladium/Kohle mit Wasserstoff reduziert. Nach dem Ende der Wasserstoffaufnahme wurde der Katalysator abfiltriert. Durch Eindampfen des Filtrats im Rotationsverdampfer bei einer Badtemperatur von maximal 30 °C wurden 2,5 g 2,4-Diaminophenoxyessigsäurenatriumsalz erhalten.

Analyse : $C_8H_9N_2O_3Na + 1,5\ H_2O$
berechnet : 41,56 % C ; 5,23 % H ; 12,11 % N ; 31,14 % O.
gefunden : 41,80 % C ; 4,94 % H ; 12,0 % N ; 31,4 % O.
IR-Spektrum (KBr) : Carbonylbanden bei 1 620 und 1 423 $cm^{-1}$.
Als Entwicklerkomponenten dienten folgende Substanzen :

E 1 : Tetraaminopyrimidinsulfat
E 2 : p-Toluylendiammoniumsulfat
E 3 : N-Methyl-p-phenylendiammoniumsulfat
E 4 : N-Ethyl-N-hydroxyethyl-p-phenylendiammoniumsulfat
E 5 : [N-{2-[N-Ethyl-N-(4-amino-3-methylphenyl)-amino]-ethyl}-methansulfonamid-sesquisulfat (Monohydrat)
E 6 : N-Ethyl-N-(2-hydroxyethyl)-1,4-phenylendiaminsulfat (Monohydrat).

Die erfindungsgemäßen Haarfärbemittel wurden in Form einer Cremeemulsion eingesetzt. Dabei wurden in eine Emulsion aus

10 Gewichtsteilen Fettalkoholen der Kettenlänge $C_{12}$-$C_{18}$,
10 Gewichtsteilen Fettalkoholsulfat (Natriumsalz) der Kettenlänge $C_{12}$-$C_{18}$,
75 Gewichtsteilen Wasser

jeweils 0,01 Mol der in der nachstehenden Tabelle aufgeführten Entwicklersubstanzen und Kupplersubstanzen eingearbeitet. Danach wurde der pH-Wert der Emulsion mittels Ammoniak auf 9,5 eingestellt und die Emulsion mit Wasser auf 100 Gewichtsteile aufgefüllt. Die oxidative Kupplung wurde mit 1 %iger Wasserstoffperoxidlösung als Oxidationsmittel durchgeführt, wobei zu 100 Gewichtsteilen der Emulsion 10 Gewichtsteile Wasserstoffperoxidlösung gegeben wurden. Die jeweilige Färbecreme mit zusätzlichem Oxidationsmittel wurde auf zu 90 % orgrautes, nicht besonders vorbehandeltes Menschenhaar aufgetragen und dort 30 Minuten belassen. nach Beendigung des Färbeprozesses wurde das Haar mit einem üblichen Haarwaschmittel ausgewaschen und anschließend getrocknet. Die dabei erhaltenen Färbungen sind nachstehender Tabelle 1 zu entnehmen.

(Siehe Tabelle 1 Seite 5 f.)

Tabelle 1

| Beispiel | a) Kuppler | b) Entwickler | mit 1%iger $H_2O_2$-Lösung erhaltener Farbstoff |
|---|---|---|---|
| 1 | K 1 | E 1 | olivgelb |
| 2 | K 1 | E 2 | schwarzblau |
| 3 | K 1 | E 3 | türkis |
| 4 | K 2 | E 4 | violettschwarz |
| 5 | K 2 | E 5 | blauschwarz |
| 6 | K 2 | E 6 | blauschwarz |
| 7 | K 2 | E 1 | dunkelgrün |
| 8 | K 2 | E 2 | dunkelviolett |

C. Natriumsalz der 3-(2,4-Diaminophenyl)-propionsäure

Herstellung der 3-(2,4-Dinitrophenyl)-propionsäure.

12 g 3-Phenylpropionsäure wurden in 48 ml conc. Schwefelsäure gelöst. Zu dieser Lösung wurde eine Mischung von 24 ml conc. Schwefelsäure und 12 ml Salpetersäure der Dichte 1,52 unter Rühren zugetropft, wobei die Temperatur unter 30 °C gehalten wurde. Nachdem 3 Stunden lang bei Raumtemperatur nachgerührt worden war, wurde auf Eis gegossen, abgesaugt, mit Wasser gewaschen und im Vakuum bei 50 °C getrocknet. Es wurden 10,6 g an 3-(2,4-Dinitrophenyl)-propionsäure vom Schmelzpunkt 113-117 °C erhalten.

Herstellung des Natriumsalzes der 3-(2,4-Diaminophenyl)-propionsäure.

Die 3-(2,4-Dinitrophenyl)-propionsäure wurde mittels Natriumhydrogencarbonat in das Natriumsalz überführt und in wäßriger Lösung entsprechend den Angaben unter A reduziert. Aus 5 g 3-(2,4-Dinitrophenyl)-propionsäure wurden 3,5 g an 3-(2,4-Diaminophenyl)-propionsaures Natrium erhalten.
Analyse : $C_8H_{11}N_2O_2Na + 2\ H_2O$
berechnet : C 45,38 % N 11,76 %
gefunden : C 45,4 % N 12,1 %
IR-Spektrum : Carboxylatbanden bei 1 560 und 1 405 $cm^{-1}$

D. Natriumsalz der 2-(2,4-Diaminophenoxy)-propionsäure

Herstellung der 2-(2,4-Dinitrophenoxy)-propionsäure.

Zu 40 ml auf − 5 °C abgekühlter Salpetersäure der Dichte 1,52 wurden bei dieser Temperatur portionsweise 9,96 g 2-Phenoxypropionsäure gegeben. Nach erfolgter Zugabe wurde noch 10 Minuten lang bei − 5 °C nachgerührt und danach entsprechend den Angaben unter C aufgearbeitet. Es wurden 14 g 2-(2,4-Dinitrophenoxy)-propionsäure vom Schmelzpunkt 167-171 °C erhalten.

Herstellung des Natriumsalzes der 2-(2,4-Diaminophenoxy)-propionsäure.

Die 2-(2,4-Dinitrophenoxy)-propionsäure wurde mittels Natriumhydrogencarbonat in das Natriumsalz überführt und in wäßriger Lösung entsprechend den Angaben unter A reduziert und isoliert. Aus 5 g 2-(2,4-Dinitrophenoxy)-propionsäure wurden 2,4 g an Natriumsalz der 2-(2,4-Diaminophenoxy)-propionsäure erhalten.
Analyse : $C_9H_{11}N_2O_3Na + 2\ H_2O$
berechnet : C 42,52 % H 5,91 %
gefunden : C 42,4 % H 5,9 %
Ir-Spektrum : Carboxylatbanden bei 1 600 und 1 412 $cm^{-1}$.

E. Natriumsalz der 2-(2,4-Diaminophenoxy)-buttersäure

Herstellung der 2-(2,4-Dinitrophenoxy)-buttersäure.

Die Synthese erfolgte analog den Angaben unter D, wobei aus 10,8 g 2-Phenoxybuttersäure 12,8 g an 2-(2,4-Dinitrophenoxy)-buttersäure vom Schmelzpunkt 120-123 °C erhalten wurden.

Herstellung des Natriumsalzes der 2-(2,4-Diaminophenoxy)-buttersäure.

Nach Überführung in das Natriumsalz wurden 5,3 g 2-(2,4-Dinitrophenoxy)-buttersäure entsprechend den Angaben unter D reduziert und aufgearbeitet. Es wurden 3,3 g an Natriumsalz der 2-(2,4-Diaminophenoxy)-buttersäure erhalten.

Analyse : $C_{10}H_{13}N_2O_3Na + 2\ H_2O$

berechnet : C ≠ 44,78 % N ≠ 10,45 %

gefunden : C ≠ 44,7 % N ≠ 10,0 %

IR-Spektrum : Carboxylatbanden bei 1 600 und 1 412 $cm^{-1}$.

Die Ausfärbungen mit den Produkten C, D, E als Kupplerkomponenten und E1 (Tetraaminopyrimidinsulfat) und E2 (p-Tolyulendiammoniumsulfat) als Entwicklersubstanzen führten zu folgenden Ergebnissen.

| Beispiel | a) Kuppler | b) Entwickler | mit 1 %iger $H_2O_2$-Lösung erhaltene Farbnuance |
|---|---|---|---|
| 9 | C | E1 | oliv |
| 10 | C | E2 | blaugrau |
| 11 | D | E1 | grautürkis |
| 12 | D | E2 | blaugrau |
| 13 | E | E1 | grautürkis |
| 14 | E | E2 | blaugrau |

**Ansprüche** (für die Vertragsstaaten : BE, CH, DE, FR, GB, IT, LI, LU, NL, SE)

1. m-Phenylendiaminderivate der Formel I,

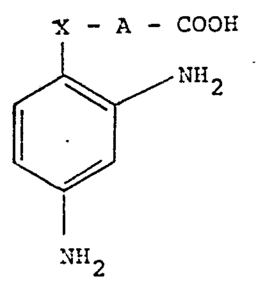

(I)

in der X ein Sauerstoffatom bedeutet oder nicht vorhanden ist, während A für einen Alkylenrest mit 1 bis 4 Kohlenstoffatomen steht.

2. Verfahren zur Herstellung der Verbindungen der Formel I nach Anspruch 1, dadurch gekennzeichnet, daß man Verbindungen der Formel II,

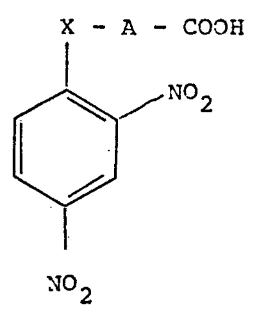

(II)

in der X und A die für die Formel I angegebene Bedeutung haben, in an sich bekannter Weise in Gegenwart eines Katalysator mit Wasserstoff umsetzt.

3. Haarfarbemittel auf Basis von Oxidationsfarbstoffen nit einem Gehalt an Verbindungen der Formel

I nach Anspruch 1 und/oder deren Ammonium-, Alkali- oder Erdalkalisalzen als Kupplerkomponenten und den in Oxidationshaarfarben üblichen Entwicklersubstanzen.

4. Haarfärbemittel nach Anspruch 3, gekennzeichnet durch einen Gehalt an Entwickler- und Kupplerkombination in einer Menge von 0,2 bis 5 Gew.-%, vorzugsweise 1 bis 3 Gew.-%, bezogen auf das gesamte Haarfärbemittel.

**Ansprüche** (für den Vertragsstaat AT)

1. Verfahren zur Herstellung der Verbindungen der Formel I

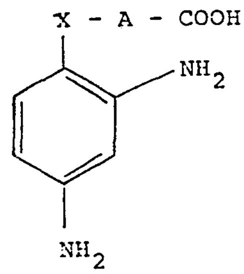

(I)

in der X ein Sauerstoffatom bedeutet oder nicht vorhanden ist, während A für einen Alkylenrest mit 1 bis 4 Kohlenstoffatomen steht, dadurch gekennzeichnet, daß man Verbindungen der Formel II

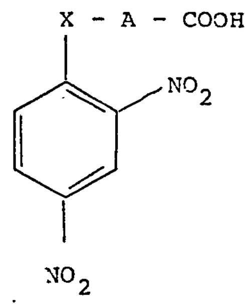

(II)

in der X und A die für die Formel I angegebene Bedeutung haben, in an sich bekannter Weise in Gegenwart eines Katalysators mit Wasserstoff umsetzt.

2. Haarfärbemittel auf Basis von Oxidationsfarbstoffen mit einem Gehalt an Verbindungen der Formel I gemäß Anspruch 1 und/oder deren Ammonium-, Alkali-oder Erdalkalisalzen als Kupplerkomponenten und den in Oxidationshaarfarben üblichen Entwicklersubstanzen.

3. Haarfärbemittel nach Anspruch 2, gekennzeichnet durch einen Gehalt an Entwickler- und Kupplerkombination in einer Menge von 0,2 bis 5 Gew.-%, vorzugsweise 1 bis 3 Gew.-%, bezogen auf das gesamte Haarfärbemittel.

**Claims** (for the Contracting States : BE, CH, DE, FR, GB, IT, LI, LU, NL, SE)

1. m-Phenylene derivatives corresponding to the following formula

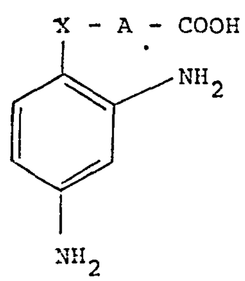

(I)

in which X is an oxygen atom or is not present whilst A represents an alkylene radical containing from 1 to 4 carbon atoms.

2. A process for producing the compounds corresponding to formula I in Claim 1, characterized in that compounds corresponding to the following formula

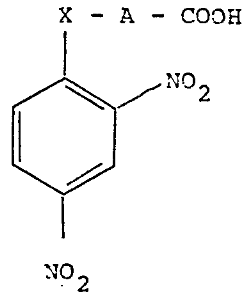

(II)

in which X and A have the same meanings as in formula I are reacted in known manner with hydrogen in the presence of a catalyst.

3. Hair dyes based on oxidation dyes containing compounds corresponding to formula I in Claim 1 and/or ammonium, alkali or alkaline earth metal salts thereof as coupler components and the developer substances normally used in oxidation hair dyes.

4. Hair dyes as claimed in Claim 3, characterized by a content of developer and coupler combination in a quantity of from 0.2 to 5 % by weight and preferably in a quantity of from 1 to 3 % by weight, based on the hair dye as a whole.

**Claims** (for the Contracting State AT)

1. A process for producing compounds corresponding to the following formula

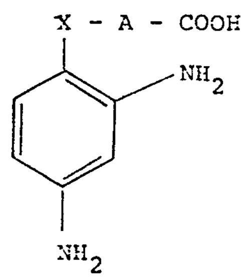

(I)

in which X is an oxygen atom or is not present whilst A represents an alkylene radical containing from 1 to 4 carbon atoms, characterized in that compounds corresponding to the following formula

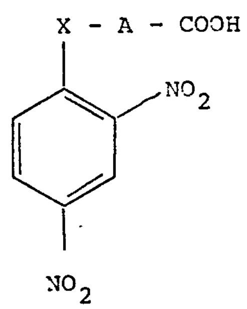

(II)

in which X and A have the meanings defined for formula I are reacted in know manner with hydrogen in the presence of a catalyst.

2. Hair dyes based on oxidation dyes containing compounds corresponding to formula I in Claim 1 and/or their ammonium, alkali or alkaline earth salts as coupler components and the developer substances normally used in oxidation hair dyes.

3. Hair dyes as claimed in Claim 2, characterized by a content of developer and coupler combination in a quantity of from 0.2 to 5 % by weight and preferably in a quantity of from 1 to 3 % by weight, based on the hair dye as a whole.

**Revendications** (pour les Etats contractants : BE, CH, DE, FR, GB, IT, LI, LU, NL, SE)

1. Dérivés de m-phénylènediamine de formule I

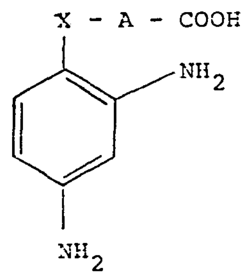

(I)

dans laquelle X signifie un atome d'oxygène ou n'est pas présent, tandis que A représente un radical alcoylène ayant 1 à 4 atomes de carbone.

2. Procédé de préparation des composés de formule I selon la revendication 1, caractérisé en ce qu'on fait réagir avec de l'hydrogène de manière connue en soi, en présence d'un catalyseur, des composés de formule II

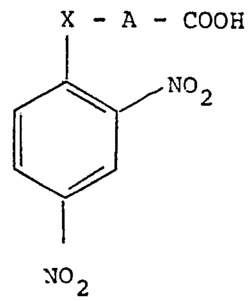

(II)

dans laquelle X et A ont la signification indiquée pour la formule I.

3. Agent de teinture des cheveux à base de colorants d'oxydation ayant une teneur en composés de formule I selon la revendication 1 et/ou en leurs sels d'ammonium, de métaux alcalins ou alcalino-terreux comme composants coupleurs et en substances de développement qui sont en usage dans la teinture oxydante des cheveux.

4. Agent de teinture des cheveux selon la revendication 3, caractérisé par une teneur en une combinaison d'agent de développement et de coupleur, en une quantité de 0,2 à 5% en poids, de préférence de 1 à 3% en poids, par rapport à l'agent de teinture de cheveux total.

**Revendications** (pour l'Etat contractant AT)

1. Procédé de préparation des composés de formule I

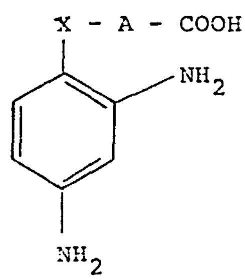

(I)

dans laquelle X signifie un atome d'oxygène ou n'est pas présent, tandis que A représente un radical alcoylène ayant 1 à 4 atomes de carbone, caractérisé en ce qu'on fait réagir avec de l'hydrogène de manière connue en soi en présence d'un catalyseur des composés de formule II :

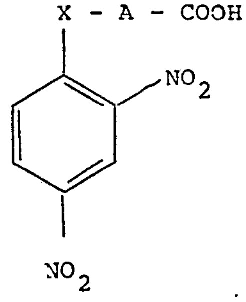

(II)

dans laquelle X et A ont la signification indiquée pour la formule I.

2. Agent de teinture des cheveux à base de colorants d'oxydation ayant une teneur en composés de formule I selon la revendication 1 et/ou en leurs sels d'ammonium, de métaux alcalins ou alcalino-terreux comme composants de couplage et en substances de développement en usage dans la teinture oxydante des cheveux.

3. Agent de teinture des cheveux selon la revendication 2, caractérisé par une teneur en une combinaison d'agent de développement et de coupleur en une quantité de 0,2 à 5 % en poids, de préférence de 1 à 3 % en poids, par rapport à l'agent de teinture des cheveux total.